# EUROPÄISCHE PATENTSCHRIFT

(11) **EP 3 541 468 B1**
(45) Veröffentlichungstag und Bekanntmachung des Hinweises auf die Patenterteilung: **15.07.2020**
(21) Anmeldenummer: 17808029.7
(22) Anmeldetag: 17.11.2017
(51) Int. Cl.: A61N 1/05

(54) **IMPLANTIERBARE ELEKTRISCHE KONTAKTANORDNUNG**
IMPLANTABLE ELECTRICAL CONTACT ARRANGEMENT
AGENCEMENT DE CONTACT ÉLECTRIQUE IMPLANTABLE

(30) Priorität: 18.11.2016 DE 102016222710
(43) Veröffentlichungstag der Anmeldung: 25.09.2019
(73) Patentinhaber: Neuroloop GmbH, 79108 Freiburg (DE)
(72) Erfinder: BORETIUS, Tim, 79108 Freiburg (DE); KIMMIG, Fabian, 79110 Freiburg (DE); HASSLER, Christina, 79276 Reute (DE); PLACHTA, Dennis, 79279 Vörstetten (DE)
(74) Vertreter: Rösler, Uwe
(86) Internationale Anmeldenummer: PCT/EP2017/079599
(87) Internationale Veröffentlichungsnummer: WO 2018/091656

(56) Entgegenhaltungen:
- EP-A1- 2 476 455
- WO-A2-2011/066552
- US-A- 5 755 759
- US-A1- 2012 109 296

## Beschreibung

### Technisches Gebiet

Die Erfindung bezieht sich auf eine implantierbare elektrische Kontaktanordnung, die wenigstens eine ansonsten vollständig aus biokompatiblem, elektrisch isolierendem Material umfasste Elektrodenkörperanordnung aufweist, mit wenigstens einer frei zugänglichen, vom dem biokompatiblen, elektrisch isolierenden Material mittel- oder unmittelbar umfassten Elektrodenoberfläche.

Gattungsgemäße Kontaktanordnungen finden bspw. Anwendung in elektromechanischen Steckverbindern, die im Allgemeinen aus zwei mechanisch lösbarfest ineinander fügbaren Komponenten zu Zwecken einer elektrischen Energie- und/oder Signalübertragung bestehen und unterliegen in Abhängigkeit ihrer Ausbildung und Anwendung spezifischen betriebssicherheitsrelevanten Anforderungen. Handelt es sich um implantierbare Steckverbinder, so müssen diese den Anforderungen für aktive, implantierbare Medizinprodukte genügen, die einem dauerhaft feuchten Milieu ausgesetzt sind und diesem für eine möglichst lange Zeitdauer unbeschadet gegenüber Feuchtigkeits- bzw. Wassereintritt in das Innere des jeweiligen Implantates standhalten sollen.

Besonders kritisch sind bei an sich bekannten implantierbaren Steckverbindern die längsseitigen Fügeabschnitte an der sich die Steckverbinderkomponenten zumeist form- und kraftschlüssig an- und ineinanderfügen. Die besondere Herausforderung bei der Konstruktion und Ausgestaltung derartiger Steckverbinder besteht darin, ein Eindringen von Wasser bzw. Feuchtigkeit in und durch die innerhalb eines Steckverbinders enthaltenen Grenzflächen zwischen Stecker- und Buchsenteil möglichst langfristig zu unterbinden, um Wasser- bzw. Feuchtigkeitskontakt mit den innerhalb eines Steckverbinders enthaltenen elektrischen Strukturen zu vermeiden. So führen Wasserkontakte an elektrisch leitenden, zumeist aus metallischem Material bestehenden Leitungs- und Elektrodenstrukturen zu irreversiblen Degradationserscheinungen und einer damit verbundenen Beeinträchtigung der elektrischen Energie- und Signalübertragungseigenschaften. Darüber hinaus vermag die Gegenwart von Wasser bzw. Feuchtigkeit Ablösungen zwischen den innerhalb des Steckverbinders enthaltenen, metallischen Strukturen und dessen unmittelbar umgebenden, zumeist aus polymeren Materialien bestehenden Oberflächen der Steckverbinderkomponenten hervorzurufen und somit die Lebensdauer derartiger Steckverbinder herabzusetzen.

### Stand der Technik

Implantierbare elektrische Steckverbinder existieren in großer Anzahl und Vielgestaltigkeit, allen bekannten Ausführungsformen ist zumindest gemein, dass Maßnahmen getroffen sind, die ein Eindringen von Feuchtigkeit, vorzugsweise in Form von Körperflüssigkeit, in den inneren, elektrisch leitenden Schichtaufbau behindern, vorzugsweise vollständig verhindern. Als Beispiele an sich bekannter elektromechanischer Steckverbinder seien die nachfolgenden Druckschriften genannt, die in nicht abschließender Form repräsentative Steckverbinder offenbaren: DE 10 2011 009 857 B4, EP 0 910 435 B1, DE 10 2012 020 260 B1, DE 20 2007 019 606 U1 sowie EP 0 811 397 B1.

In der Druckschrift US 2012/0109296 A1 ist eine implantierbare Retinaanordnung mit einer Reihe von integrierten Sensoren und mikroelektronischen Bauelementen beschrieben, die in Schichtbauweise gefertigt sind.

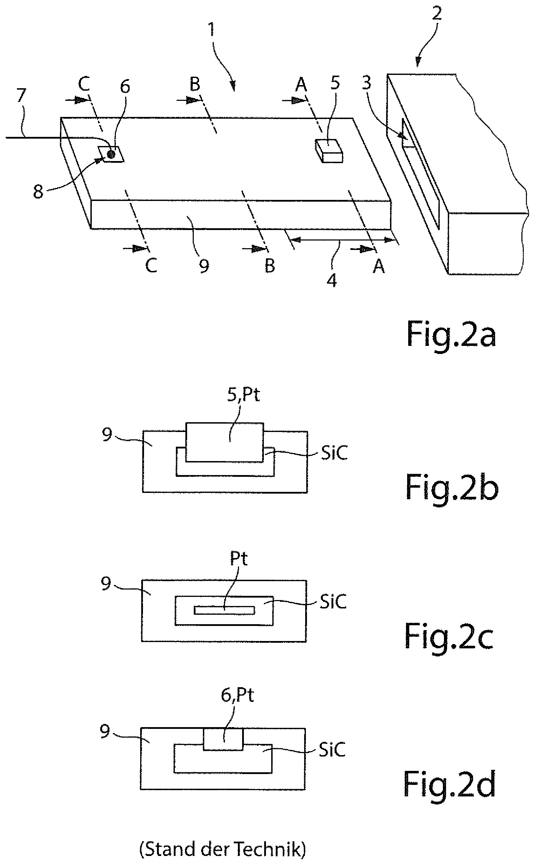

Ein an sich bekannter implantierbarer Steckverbinder ist in Figur 2a illustriert, mit einem Steckerteil 1 und einem Buchsenteil 2. Das Steckerteil 1 ist in längs seines vorderen Fügeabschnittes 4 vollständig in die Einschuböffnung 3 des Buchsenteils 2 einführbar. Im Bereich des Fügeabschnittes 4 ist aus Gründen einer einfacheren Darstellung und Erläuterung lediglich ein Elektrodenkörper 5 angebracht, der vorzugsweise erhaben über die Oberfläche des Steckerteils 1 ausgebildet ist. Typischerweise weisen reelle Steckverbinder eine Vielzahl derartiger Elektrodenkörper auf. Der Elektrodenkörper 5 kontaktiert im eingefügten Zustand des Steckerteils 1 innerhalb des Buchsenteils 2 eine entsprechend innenseitig im Buchsenteil 2 angebrachte Gegenelektrode (nicht dargestellt) zu Zwecken einer elektrischen Signal- bzw. Energieübertragung.

Das Steckerteil 1 weist darüber hinaus in einem dem Fügeabschnitt 4 abgewandten Steckerteilbereich eine Kontaktelektrodenfläche 6 auf, an der eine externe elektrische Zuleitung 7, beispielsweise im Wege einer Lötverbindung 8 angebracht ist und für eine externe elektrische Signal- und Energieversorgung sorgt.

Zur Illustration des inneren Aufbaus des Steckerteils 1 sind in den Figuren 2b, c und d Querschnitte durch das Steckerteil 1 längs der in Figur 2a eingetragenen Schnitte AA, BB sowie CC dargestellt.

Das Steckerteil 1 weist ein mit Ausnahme der Kontaktelektrodenfläche 6 sowie des Elektrodenkörpers 5 vollständig umfassendes Gehäuse 9 auf, das aus einem biokompatiblen, vorzugsweise elastischen, elektrisch nicht leitenden Polymer besteht.

Der Elektrodenkörper 5 und die Kontaktelektrodenfläche 6 sind aus Platin gefertigt und im Inneren des Steckerteils 1 einstückig miteinander verbunden. Das Elektrodenmaterial Platin Pt ist auf einem Substratträger 10 aus Siliziumcarbid aufgebracht, wie dies aus den Schnittdarstellungen gemäß der Figuren 2b, c und d zu entnehmen ist.

Gleichwohl die Verbindung zwischen dem SiC-Substratträger 10 und dem Platin-Elektrodenmaterial eine bevorzugte Bondverbindung darstellt, zeigt sich insbesondere bei zunehmender Betriebs- und Lebensdauer derartiger implantierter Steckverbinder eine Degradation an der Grenzfläche zwischen Platin und SiC. Oxidations- und Hydrationsprozesse, bedingt durch eine am Elektrodenmaterial kontinuierlich oder pulsatil anliegende, elektrische Spannung tragen dazu bei, dass sich Änderungen in der metallischen Gitterkonstante ausbilden, die zu Deformationen an der Oberfläche der Platinelektrode führen, wodurch Ablöseerscheinungen an der Grenzfläche zwischen Platin und Siliziumcarbid auftreten. Derartige Prozesse führen unweigerlich zum irreversiblen Funktionsverlust des implantierbaren Steckverbinders.

Aus der Druckschrift DE 10 2011 078 982 A1 ist eine implantierbare Nervenelektrode zu entnehmen, die ein aus medizinischen Silikon bestehendes Trägersubstrat aufweist, in das Leiterbahnen integriert sind, die jeweils Elektrodenkontakte und Anschlusskontakte verbinden. Die Leiterbahnen, sowie die Elektrodenkontakte und Anschlusskontakte sind aus rostfreiem Stahl oder Platin hergestellt.

Die Druckschrift DE 10 2014 014 943 A1 offenbart eine implantierbare Elektrodenanordnung, bei der innerhalb eines aus biokompatiblen Polymer bestehenden Trägersubstrats speziell strukturierte Elektroden eingebettet sind, die jeweils an die Trägersubstratoberfläche mündende Elektrodenkontaktflächen besitzen und ansonsten wenigstens eine von der Trägersubstratoberfläche abgewandte Elektrodenoberfläche aufweisen, die keinen oder nur einen sehr erschwerten Zugang zum intrakorporalen feuchten Milieu besitzt.

Die Druckschrift US 2011/0034977 A1 beschreibt eine implantierbare Elektrodenanordnung mit einer Vielzahl auf einem, teilelastisch verformbaren Tragekörper angebrachten Elektroden, die allesamt mit innerhalb des Tragekörpers integrierten Kontrolleinheiten verbunden sind.

### Darstellung der Erfindung

Der Erfindung liegt die Aufgabe zugrunde eine implantierbare elektrische Kontaktanordnung, die wenigstens eine ansonsten vollständig aus biokompatiblen, elektrisch isolierendem Material umfasste Elektrodenkörperanordnung aufweist, mit wenigstens einer frei zugänglichen, von dem biokompatiblen, elektrisch isolierendem Material mittel- oder unmittelbar umfassten Elektrodenoberfläche derart auszubilden, dass betriebsbedingte Degradationserscheinungen, wie vorstehend erläutert nicht, oder nur in einem vernachlässigbar geringen Umfang auftreten, so dass die Lebensdauer gattungsgemäßer Kontaktanordnungen gesteigert wird. Darüber hinaus sollen die lösungsgemäßen Maßnahmen dazu beitragen, die Resistenz implantierbarer elektrischer Kontaktanordnungen gegen Feuchtigkeitseintritt aufgrund des vorherrschenden intrakorporalen feuchten Milieus zu verbessern.

Die Lösung der der Erfindung zugrundeliegenden Aufgabe ist im Anspruch 1 angegeben. Den Erfindungsgedanken in vorteilhafter Weise weiterbildende Merkmale sind Gegenstand der Unteransprüche sowie der weiteren Beschreibung insbesondere unter Bezugnahme auf die Ausführungsbeispiele zu entnehmen.

Lösungsgemäß zeichnet sich eine implantierbare elektrische Kontaktanordnung gemäß den Merkmalen des Oberbegriffes des Anspruches 1 dadurch aus, dass die Elektrodenkörperanordnung einen stapelförmigen Schichtverbund aufweist, der zumindest eine über eine Diffusionssperrschicht mit einer Iridiumschicht verbundene Goldschicht vorsieht. Der stapelförmige Schichtverbund ist ansonsten vollständig mit einer SiC-Schicht ummantelt, mit Ausnahme wenigstens eines, dem Schichtverbund abgewandten Oberflächenbereiches der Iridiumschicht. Die SiC-Schicht wiederum weist eine dem stapelförmigen Schichtverbund abgewandte SiC-Schichtoberfläche auf, an der das biokompatible, elektrisch isolierende Material mittel- oder unmittelbar angrenzt.

Durch Vorsehen einer zwischen der Goldschicht und der Iridiumschicht eingebrachten Diffusionssperrschicht, die aus einem Übergangsmetall besteht, deren Gitterkonstante kleiner als die Gitterkonstante von Gold jedoch größer als die Gitterkonstante von Iridium gewählt ist, wird ein stapelförmiger Schichtverbund realisiert, an dessen Grenzflächen überwiegend hohe kohäsive Bindungskräfte wirken, d.h. der Bindungsanteil der kohäsiven Bindungskräfte zwischen den Grenzflächen beträgt wenigstens 70%, vorzugsweise wenigstens 80%, insbesondere vorzugsweise wenigstens 90%. Die übrigen Bindungskräfte beruhen bspw. auf kovalenten oder adhäsiven Bindungen o.ä..

In einer besonders bevorzugten Ausführungsform wird als Diffusionssperrschicht Platin verwendet, ebenso ist jedoch auch der Einsatz von Titan möglich. Der lösungsgemäße stapelförmige Schichtverbund, vorzugsweise bestehend aus der Schichtabfolge SiC, Au, Pt, Ir weist optimal aufeinander abgestimmte Gitterkonstanten auf, durch die sich ein inniger, auf starken kohäsiven Bindungskräften beruhender, stapelförmiger Schichtverbund ausbildet. Dies spiegelt sich in den Gitterkonstanten der nachfolgenden bevorzugten Schichtabfolge wider: SiC: 4,36 Å, Au: 4,07 Å, Pt: 3,92 Å, Ir: 3,83 Å.

Durch die unmittelbare Schichtabfolge der SiC-Schicht und der darauf befindlichen Goldschicht kann die Ausbildung von Carbiden, die der Ausbildung kohäsiver Bindungskräfte entgegenwirken, ausgeschlossen werden. Zudem ist der Schichtverbund aus SiC und Gold temperaturstabil bis Temperaturen von maximal 550°C.

Sowohl zum Zwecke einer möglichst optimalen Anpassung der Gitterkonstanten innerhalb des schichtförmigen Stapelverbundes, wie vorstehend erwähnt, als auch zur Verhinderung einer Diffusion von Gold in die angrenzende Ir-Schicht dient die Platin- bzw. Titanschicht als Diffusionssperrschicht und verfügt vorzugsweise lediglich über eine Schichtdicke von etwa 20 bis 30 Nanometer.

In einer bevorzugten Ausführungsform der lösungsgemäß ausgebildeten elektrischen Kontaktanordnung entspricht der wenigstens eine nicht von der SiC-Schicht bedeckte Oberflächenbereich der Ir-Schicht der wenigstens einen Elektrodenoberfläche der Kontaktanordnung. In vorteilhafter Weiterbildung ist es gleichfalls möglich, zusätzlich auf der frei zugänglichen Iridiumschicht eine Iridiumoxidschicht aufzubringen, die der wenigstens einen Elektrodenoberfläche der Kontaktanordnung entspricht.

Mit Ausnahme der jeweils wenigstens einen frei zugänglichen Elektrodenoberfläche ist die gesamte Elektrodenkörperanordnung von dem biokompatiblen, elektrisch isolierenden Polymer umgeben, das vorzugsweise ein Polyimid, Liquid Crystal Polymer (LCP), Parylen oder PDMS ist.

Der sich durch signifikant hohe kohäsive Bindungskräfte zwischen den Schichten auszeichnende, stapelförmige Schichtverbund eignet sich in besonders vorteilhafter Weise für den Aufbau eines Stecker- und/oder Buchsenteils eines implantierbaren elektrischen Steckverbinders, der sich dadurch auszeichnet, dass das die wenigstens eine Elektrodenanordnung umfassende biokompatible, elektrisch isolierende Material die Oberfläche eines Körpers begrenzt, an der mittel- oder unmittelbar die wenigstens eine frei zugängliche Elektrodenoberfläche der Elektrodenkörperanordnung angrenzt.

Im Falle eines Steckerteils ist wenigstens eine weitere frei zugängliche Elektrodenoberfläche an der Oberfläche des Körpers vorgesehen, über die vorzugsweise eine externe Signal- bzw. elektrische Energieversorgung erfolgt. Die wenigstens zwei Elektrodenoberflächen sind über eine innerhalb des Körpers verlaufende elektrische Verbindung elektrisch verbunden.

In einer bevorzugten Ausführungsform sind beide frei zugänglichen Elektrodenoberflächen mittel- oder unmittelbar mit einer elektrischen Verbindung aus Gold verbunden, die einstückig mit der Goldschicht der lösungsgemäßen Elektrodenkörperanordnung einseitig verbunden und ansonsten von der SiC-Schicht vollständig umfasst ist, die ihrerseits von dem biokompatiblen, elektrisch isolierenden Material des Körpers umgeben ist.

Im Falle eines Buchsenteils, in dem nicht notwendigerweise wenigstens eine weitere frei zugängliche Elektrodenoberfläche vorgesehen sein muss, ist die wenigstens eine frei zugängliche Elektrodenoberfläche innerhalb des Körpers mit einer elektrischen Verbindung elektrisch verbunden, die mit einem beliebigen intrakorporal platzierten Verbraucher verbunden ist.

Selbstverständlich lässt sich die lösungsgemäß ausgebildete implantierbare elektrische Kontaktanordnung auch in anderen elektrischen Komponenten und Systemen integrieren und einsetzen, die einem fortwährenden feuchten Milieu ausgesetzt sind und deren Zugänglichkeit zumindest erschwert ist, sodass eine möglichst lange Systemlebensdauer für einen ansonsten autarken Betrieb gefordert ist. Beispielsweise eignet sich die Kontaktanordnung auch als Stimulationsanordnung zur Applikation elektrischer Impulse an intrakorporale Gewebebereiche. Hierbei dient die frei zugängliche Kontaktelektrodenfläche, vorzugsweise in Form der vorstehend erwähnten Iridiumoxidschicht, als aktive Stimulationselektrode, die in mittel- oder unmittelbaren körperlichen und somit auch elektrisch leitfähigen Kontakt an einen physiologischen Gewebebereich, bspw. längs eines Nerven oder Muskels, zu dessen Stimulation gebracht wird.

Die Iridiumoxidschicht verfügt überdies über eine hinreichend poröse Elektrodenoberfläche, so dass die elektrochemisch aktive Elektrodenfläche um ein vielfaches größer als die geometrische Elektrodenfläche ist. Dies trägt u.a. zu einer reduzierten Impedanz der Elektrodenfläche bei und erhöht die maximale Ladungsinjektionsdichte drastisch.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1a, b, c: Schnittdarstellungen durch einen Steckerteil einer implantierbaren Steckverbinderanordnung sowie
- Fig. 2a: schematisierte Draufsicht auf einen implantierbaren Steckverbinder nach Stand der Technik sowie
- Fig. 2b, c, d: Schnittdarstellungen längs der in Figur 2a illustrierten Schnitte AA, BB, CC

### Wege zur Ausführung der Erfindung, gewerbliche Verwendbarkeit

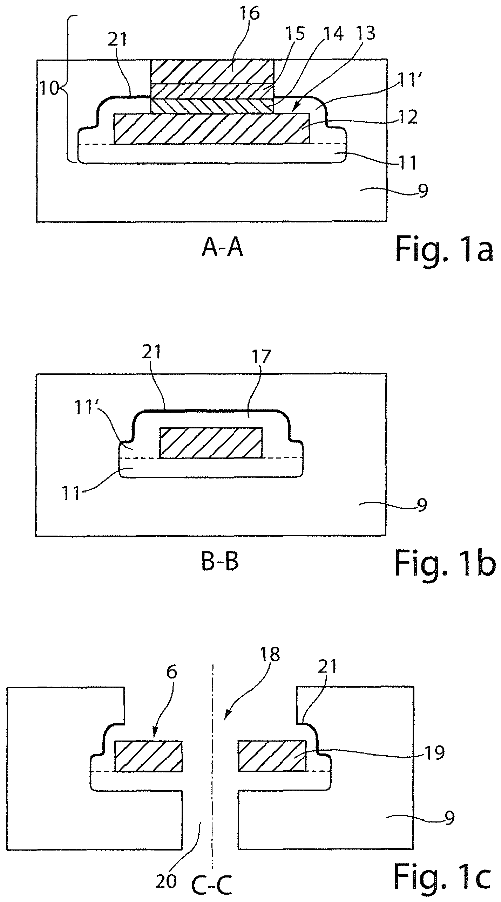

Ausgehend von der in Figur 2a illustrierten Darstellung eines Steckerteils 1, der lediglich aus Gründen der Übersichtlichkeit einen einzigen Elektrodenkörper 5 sowie eine mit diesem elektrisch verbundene Kontaktelektrodenfläche 6 besitzt, - üblicherweise befindet sich eine Vielzahl derartiger Elektrodenkörper 5 sowie dazu korrespondierende Kontaktelektrodenflächen 6 auf einem an sich bekannten Steckerteil 1 - sei im weiteren angenommen, dass sich ein lösungsgemäß ausgebildeter Steckerteil äußerlich von der bekannten Form eines Steckerteils nicht ersichtlich unterscheidet. Die lösungsgemäße Ausbildung spiegelt sich in der Anordnung und im Aufbau der im Inneren des Steckerteils 1 integrierten Elektrodenkörperanordnung wider, die unter Bezugnahme auf Figur 1a im Weiteren näher erläutert wird.

Figur 1a zeigt eine Schnittdarstellung längs einer Schnittebene AA eines lösungsgemäß ausgebildeten Steckerteils 1, gemäß des in Figur 2a angedeuteten Schnittes AA. Innerhalb eines aus biokompatiblen, elektrisch isolierenden Polymer bestehenden Gehäuses 9 ist eine Elektrodenanordnung 10 integriert, die sich durch den folgenden Schichtaufbau auszeichnet:
Auf einer als Trägersubstrat 11 dienenden SiC-Schicht ist eine Goldschicht 12 abgeschieden, auf deren dem Trägersubstrat 11 abgewandten Goldschichtoberfläche 13 eine Diffusionssperrschicht 14 in Form einer nur wenige 10 nm dicken Platinschicht abgeschieden ist. Die Diffusionssperrschicht 14 deckt lediglich einen Teilbereich der Goldschichtoberfläche 13 derart ab, so dass die Diffusionssperrschicht 14 in Projektion auf die Sicht von einer Goldschichtoberfläche 13 lateral umgeben bleibt. Die Anordnung und Ausbildung der drei aufeinander abgeschiedenen Schichten 14, 15 und 16 ist derart vorgenommen, so dass die drei Schichten längs der vertikalen Stapelrichtung jeweils bündig miteinander abschließen.

Sowohl die Goldschicht 12 als auch die Diffusionssperrschicht 14 sowie die angrenzende Iridiumschicht 15 längs zumindest einer Teilschichtdicke, werden seitlich von einer zusätzlichen SiC-Schicht 11' hermetisch umfasst, die mit der als Trägersubstrat 11 dienenden SiC-Schicht einstückig verbunden ist. Vorzugsweise wird auf die obere SiC-Schicht 11' noch eine weitere Adhäsionsfördernde Schicht 21 aus Diamond Like Carbon (DLC) abgeschieden, die mit der SiC-Schicht 11' bündig schließt.

Die Elektrodenkörperanordnung 10 setzt sich somit aus folgendem Schichtverbund zusammen: SiC-Schicht 11 sowie 11', DLC-Schicht 21, Goldschicht 12, Diffusionssperrschicht 14, Iridiumschicht 15 sowie Iridiumoxidschicht 16.

Die gesamte Elektrodenkörperanordnung 10 ist in einem aus biokompatiblen, elektrisch isolierenden Polymer, vorzugsweise Polyimid, Liquid Crystal Polymer, Parylen oder PDMS eingebettet, vorzugsweise ragt ein Teil der Iridiumoxidschicht 16 aus dem Polymer-Gehäuse 9 empor und bildet die Kontaktoberfläche des Elektrodenkörpers 5, die nach entsprechender Einfügung des Steckerteils 1 in das Buchsenteil 2 eine innerhalb des Buchsenteils 2 vorgesehene Gegenelektrodenoberfläche kontaktiert.

Wie bereits erwähnt kann anstelle von Platin auch Titan als Diffusionssperrschicht 14 eingesetzt werden.

Zur elektrischen Verbindung der Iridiumoxidschicht 16, die dem in Figur 2a bezeichneten Elektrodenkörper 5 entspricht, mit der Kontaktelektrodenfläche 6 dient eine im Inneren des Gehäuses 9 verlaufende Goldverbindungsleitung 17, die einseitig einstückig mit der Goldschicht 12 der Elektrodenkörperanordnung 10 verbunden ist. Die Goldverbindungsleitung 17 ist mit einer SiC-Schicht 11/11' vollständig ummantelt und gegenüber dem Polymer-Gehäuse 9 hermetisch abgegrenzt, siehe Schnitt BB in Fig. 1b.

Im Bereich der Kontaktelektrodenfläche 6, siehe Schnittdarstellung CC in Fig. 1c ist im Polymergehäuse 9 eine Ausnehmung 18 eingebracht, die einen freien Zugang zur Oberfläche einer Goldkontaktschicht 19 darstellt, die einstückig mit der Goldverbindungsleitung 17 verbunden ist. Die Goldkontaktschicht 19 weist eine von der Goldverbindungsleitung 17 abweichende Form, Dicke und Größe auf und ist in geeigneter Weise zur Ausbildung einer Kontaktelektrodenfläche angepasst.
Insbesondere ist die frei zugängliche Kontaktelektrodenfläche 6 vollständig von der SiC-Schicht 11' lateral eingerahmt bzw. umfasst, auf der die DLC-Schicht 21 abgeschieden ist. Zu Zwecken einer dauerhaften mechanischen Verbindung zu einer elektrischen Zuleitung weist der Steckerteil am Ort der Kontaktelektrodenfläche 6 eine lokale, vollständige Durchdringung in Form einer Bohrung 20 auf.

Sowohl in der Anwendung der lösungsgemäßen implantierbaren, elektrischen Kontaktanordnung als Steckerteil, wie vorstehend erläutert, als auch in anderen implantierbaren Systemen, wie bspw. in Form einer Simulationselektrodenanordnung zur elektrischen Stimulation intrakorporaler Körperbereiche, ist der in Figur 1a illustrierte Schichtverbund, d.h. SiC-DLC-Au-Pt-Ir-IrOₓ sehr vorteilhaft, um ein Eindringen von Wasser zwischen den einzelnen Schichten zu vermeiden.

### Bezugszeichenliste

- 1: Steckerteilt
- 2: Buchsenteil
- 3: Einschuböffnung
- 4: Fügeabschnitt
- 5: Elektrodenkörper
- 6: Kontaktelektrodenfläche
- 7: Elektrische Zuleitung
- 8: Lötverbindung
- 9: Gehäuse
- 10: Elektrodenkörperanordnung
- 11: SiC-Schicht
- 11': SiC-Schicht
- 12: Goldschicht
- 13: Oberfläche der Goldschicht
- 14: Diffusionssperrschicht
- 15: Iridiumschicht
- 16: Iridiumoxidschicht
- 17: Goldverbindungsleitung
- 18: Ausnehmung
- 19: Goldkontaktschicht
- 20: Bohrung
- 21: DLC-Schicht

## Patentansprüche

1. Implantierbare elektrische Kontaktanordnung, die wenigstens eine ansonsten vollständig aus biokompatiblem, elektrisch isolierendem Material umfasste Elektrodenkörperanordnung (10) aufweist, mit wenigstens einer frei zugänglichen, vom dem biokompatiblen, elektrisch isolierenden Material mittel- oder unmittelbar umfassten Elektrodenoberfläche (6),
wobei die Elektrodenkörperanordnung (10) einen stapelförmigen Schichtverbund aufweist, **dadurch gekennzeichnet, dass** der stapelförmige Schichtverbund zumindest eine über eine Diffusionssperrschicht (14) mit einer Iridiumschicht (15) verbundene Goldschicht (12) vorsieht,
dass der stapelförmige Schichtverbund ansonsten vollständig mit einer SiC-Schicht (11, 11') ummantelt ist, mit Ausnahme wenigstens eines, dem Schichtverbund abgewandten Oberflächenbereiches der Iridiumschicht (15), und
dass die SiC-Schicht (11, 11') eine dem stapelförmigen Schichtverbund abgewandte SiC-Schichtoberfläche aufweist, an der das biokompatible, elektrisch isolierende Material mittel- oder unmittelbar angrenzt.

2. Kontaktanordnung nach Anspruch 1,
**dadurch gekennzeichnet, dass** der wenigstens eine nicht von der SiC-Schicht (11, 11') bedeckte Oberflächenbereich der Iridiumschicht (15) der wenigstens einen Elektrodenoberfläche (6) entspricht.

3. Kontaktanordnung nach Anspruch 1,
**dadurch gekennzeichnet, dass** zumindest auf dem wenigstens einen nicht von der SiC-Schicht (11, 11') bedeckten Oberflächenbereich der Iridiumschicht (15) eine Iridiumoxidschicht (16) aufgebracht ist, die eine der Iridiumschicht (15) abgewandte und der wenigstens einen Elektrodenoberfläche (6) entsprechenden frei zugänglichen Oberfläche besitzt.

4. Kontaktanordnung nach einem der Ansprüche 1 bis 3,
**dadurch gekennzeichnet, dass** das biokompatible, elektrisch isolierende Material aus einem Polymer besteht.

5. Kontaktanordnung nach Anspruch 4,
**dadurch gekennzeichnet, dass** das Polymer eines der nachstehenden Polymer ist: Polyimid, Liquid crystal polymer (LCP), Parylen oder PDMS.

6. Kontaktanordnung nach einem der Ansprüche 1 bis 5,
**dadurch gekennzeichnet, dass** die Diffusionssperrschicht (14) aus einem Übergangsmetall besteht, das eine Gitterkonstante besitzt, die kleiner als die Gitterkonstante von Gold und größer als die Gitterkonstante als Iridium ist.

7. Kontaktanordnung nach einem der Ansprüche 1 bis 6,
**dadurch gekennzeichnet, dass** die Diffusionssperrschicht (14) eine Platin- oder Titanschicht ist.

8. Kontaktanordnung nach einem der Ansprüche 1 bis 7,
**dadurch gekennzeichnet, dass** der von der SiC-Schicht (11, 11') umfasste stapelförmige Schichtverbund zumindest folgende Grenzflächen aufweist: SiC/ Au, Au/Pt, Pt/Ir, und
dass der Verbund zwischen den Grenzflächen überwiegend durch kohäsive Bindungskräfte charakterisiert ist.

9. Kontaktanordnung nach Anspruch 8,
**dadurch gekennzeichnet, dass** zwischen den Grenzflächen ein Bindungsanteil von wenigstens 70 % an kohäsiven Bindungskräften vorherrscht.

10. Kontaktanordnung nach einem der Ansprüche 1 bis 9,
**dadurch gekennzeichnet, dass** zumindest die Diffusionssperrschicht (14) hermetisch von der Gold-, SiC- und Ir-Schicht umgeben ist.

11. Kontaktanordnung nach einem der Ansprüche 1 bis 10,
**dadurch gekennzeichnet, dass** die dem stapelförmigen Schichtverbund abgewandte SiC-Schichtoberfläche zumindest bereichsweise mit einer Adhäsionsfördernden Schicht (21) aus Diamond Like Carbon (DLC) beschichtet ist

12. Stecker- oder Buchsenteil (1,2) eines implantierbaren elektrischen Steckverbinders, der wenigstens eine Kontaktanordnung nach einem der Ansprüche 1 bis 11 umfasst,
**dadurch gekennzeichnet, dass** die wenigstens eine Elektrodenkörperanordnung (10) umfassende biokompatible, elektrisch isolierende Material die Oberfläche eines Körpers begrenzt, an der mittel- oder unmittelbar die wenigstens eine frei zugängliche Elektrodenoberfläche (6) der Elektrodenkörperanordnung sowie wenigstens eine weitere frei zugängliche Elektrodenoberfläche angrenzen, und dass beide Elektrodenoberflächen über eine innerhalb des Körpers verlaufende elektrische Verbindung elektrisch verbunden sind.

13. Stecker- oder Buchsenteil nach Anspruch 12,
**dadurch gekennzeichnet, dass** die elektrische Verbindung mit der Goldschicht (12) der Elektrodenkörperanordnung (10) verbunden ist,
dass die elektrische Verbindung von einer SiC-Schicht (11, 11') umfasst ist, die von dem biokompatiblen, elektrisch isolierenden Material des Körpers umgeben ist, und dass die weitere frei zugängliche Elektrodenoberfläche (6) eine Oberfläche eines Elektrodenkörpers (5) ist, der ansonsten von der SiC-Schicht (11, 11') umfasst ist.

14. Stecker - oder Buchsenteil nach Anspruch 13,
**dadurch gekennzeichnet, dass** der Elektrodenkörper (5), die elektrische Verbindung sowie die Goldschicht (12) der Elektrodenkörperanordnung einstückig verbunden sind und aus Gold bestehen.

## Claims

1. Implantable electrical contact assembly comprising at least one electrode body assembly (10) otherwise completely made of a biocompatible, electrically insulating material, with at least one electrode surface (6) directly or indirectly surrounded by the biocompatible, electrically insulating material
wherein
the electrode body assembly (10) comprises a stack-shaped layered composite, **characterised in that** the stack-shaped layered composite envisages at least one diffusion barrier layer (14) with a gold layer (12) attached to an iridium layer (15),
**in that** the stack-shaped layered composite is otherwise completely covered with an SiC layer (11, 11') with the exception of at least one area of the surface of the iridium layer (15) directed away from the layered composite and
**in that** the SiC layer (11, 11') comprises a SiC layer surface which is directed away from the stack-shaped layered composite on which the biocompatible, electrically insulating material direct or indirectly abuts.

2. Contact assembly according to claim 1
**characterised in that** the at least one area of the surface of the iridium layer (15) not covered by the SiC layer (11, 11') corresponds to the at least one electrode surface (6).

3. Contact assembly according to claim 1
**characterised in that** at least on the at least one area of the surface of the iridium layer (15) covered by the SiC layer (11, 11'), an iridium oxide layer (16) is applied, which has a freely accessible area directed away from the iridium layer (15) and corresponding to the at least one electrode surface (6).

4. Contact assembly according to any one of claims 1 to 3
**characterised in that** the biocompatible, electrically insulating material consists of a polymer.

5. Contact assembly according to claim 3
**characterised in that** the polymer is one of the following polymers: polyimide, liquid crystal polymer (LCP), parylene of PDMS.

6. Contact assembly according to any one of claims 1 to 5
**characterised in that** the diffusion barrier layer (14) consists of a transition metal that has a lattice constant that is smaller than the lattice constant of gold and larger than the lattice constant of iridium.

7. Contact assembly according to any one of claims 1 to 6, **characterised in that** the diffusion barrier layer (14) is a platinum or titanium layer.

8. Contact assembly according to any one of claims 1 to 7
**characterised in that** the stack-shaped layered composite covered by the SiC layer (11, 11') comprises at least the following interfaces: SiC/Au, AU/Pt, Pt/Ir and
**in that** the composite between the interfaces is predominantly **characterised by** cohesive binding forces.

9. Contact assembly according to claim 8
**characterised in that** between the interfaces a binding proportion of at least 70 % cohesive binding forces predominates.

10. Contact assembly according to any one of claims 1 to 9
**characterised in that** at least the diffusion barrier layer (14) is hermetically surrounded by the gold, SiC and Ir layer.

11. Contact assembly according to any one of claims 1 to 10
**characterised in that** the SiC layer surface directed away from the stack-shaped layered composite is at least in sections coated with an adhesion-promoting layer (21) of diamond-like carbon (DLC).

12. Plug or socket component (1, 2) of an implantable electrical plug connector which comprises at least one contact assembly in accordance with any one of claims 1 to 11,
**characterised in that** the at least one electrode body assembly (10) made of a biocompatible, electrically insulating material delimits the surface of a body, on which a freely accessible electrode surface (6) of the electrode body assembly as well as at least one further freely accessible electrode surface directly or indirectly abut,
and
**in that** both electrode surfaces are electrically connected by way of an electrical connection extending within the body.

13. Plug or socket component according to claim 12,
**characterised in that** the electrical connection is connected to the gold layer (12) of the electrode body assembly (10), **in that** the electrical connection is surrounded by an SiC layer (11, 11') which is covered by the biocompatible, electrically insulating material of the body, and
**in that** the other, freely accessible electrode surface (6) is a surface of an electrode body (5) which is otherwise surrounded by the SiC layer (11, 11').

14. Plug or socket component according to claim 13
**characterised in that** the electrode body (5), the electrical connection as well as the gold layer (12) of the electrode body assembly are connected in one piece and are made of gold.

## Revendications

1. Système de contact électrique implantable, qui comporte au moins un système de corps d'électrode (10) entouré sinon complètement d'un matériau biocompatible, électro-isolant avec au moins une surface d'électrode (6) librement accessible, entourée indirectement ou directement d'un matériau,
sachant que
le système de corps d'électrode (10) comporte un matériau composite stratifié en empilement, **caractérisé en ce que** le matériau composite stratifié en empilement prévoit au moins une couche d'or (12) reliée par une couche antidiffusion (14) à une couche d'iridium (15),
**en ce que** le matériau composite stratifié en empilement est enveloppé sinon complètement d'une couche de carbone de silicium (SiC) (11, 11') à l'exception au moins d'une zone de surface de la couche d'iridium (15) opposée au matériau composite stratifié et
**en ce que** la couche de SiC (11, 11') comporte une surface de couche de SiC opposée au matériau composite stratifié en empilement à laquelle vient se juxtaposer indirectement ou directement le matériau biocompatible, électro-isolant.

2. Système de contact selon la revendication 1,
**caractérisé en ce qu'au** moins une zone de surface de la couche d'iridium (15) non recouverte par la couche SiC (11, 11') correspond au moins à une surface d'électrode (6).

3. Système de contact selon la revendication 1,
**caractérisé en ce qu'**au moins sur une zone de surface de la couche d'iridium (15) non recouverte par la couche de SiC (11, 11') est appliquée une couche d'oxyde d'iridium (16) qui possède une surface librement accessible opposée à la couche d'iridium (15) et correspondant au moins à une surface d'électrode (6).

4. Système de contact selon l'une quelconque des revendications 1 à 3,
**caractérisé en ce que** le matériau biocompatible, électro-isolant est composé d'un polymère.

5. Système de contact selon la revendication 4,
**caractérisé en ce que** le polymère est un des polymères suivants : polyimide, polymère à cristaux liquides (PCL), parylène ou polydiméthylsiloxane (PDMS).

6. Système de contact selon l'une quelconque des revendications 1 à 5,
**caractérisé en ce que** la couche antidiffusion (14) est composée d'un métal de transition, qui possède une constante de réseau, qui est plus petite que la constante de réseau de l'or et plus grande que la constante de réseau de l'iridium.

7. Système de contact selon l'une quelconque des revendications 1 à 6,
**caractérisé en ce que** la couche antidiffusion (14) est une couche de platine ou de titane.

8. Système de contact selon l'une quelconque des revendications 1 à 7,
**caractérisé en ce que** le matériau composite stratifié en empilement entouré de la couche de SiC (11, 11') comporte au moins les surfaces limites suivantes : SiC/Au, Au/Pt, Pt/Ir, et
**en ce que** le matériau composite entre les surfaces limites est surtout **caractérisé par** des forces de liaison cohésives.

9. Système de contact selon la revendication 8,
**caractérisé en ce qu'**entre les surfaces limites, il prédomine une part de liaison d'au moins 70 % en forces de liaison cohésives.

10. Système de contact selon l'une quelconque des revendications 1 à 9,
**caractérisé en ce qu'**au moins la couche antidiffusion (14) est entourée hermétiquement de la couche d'or, de SiC et d'Ir.

11. Système de contact selon l'une quelconque des revendications 1 à 10,
**caractérisé en ce que** la surface de couche de SiC opposée au matériau composite stratifié en empilement est revêtue au moins par endroits d'une couche (21) favorisant l'adhérence de carbone Adamantin (DLC).

12. Partie de connecteur mâle ou femelle (1, 2) d'un connecteur électrique implantable, qui comprend au moins un système de contact selon l'une quelconque des revendications 1 à 11,
**caractérisée en ce qu'au** moins un matériau biocompatible électro-isolant entourant un système de corps d'électrode (10) délimite la surface d'un corps sur laquelle viennent se juxtaposer indirectement ou directement au moins une surface d'électrode (6) librement accessible du système de corps d'électrode ainsi qu'au moins une autre surface d'électrode librement accessible et
**en ce que** les surfaces d'électrodes sont électriquement reliées par une liaison électrique passant à l'intérieur du corps.

13. Partie de connecteur mâle ou femelle selon la revendication 12,
**caractérisée en ce que** la liaison électrique est reliée à la couche d'or (12) du système de corps d'électrode (10),
**en ce que** la liaison électrique est entourée d'une couche de SiC (11, 11'), qui est entourée du matériau biocompatible électro-isolant du corps et
**en ce que** l'autre surface d'électrode (6) librement accessible est une surface d'un corps d'électrode (5), qui est sinon entourée de la couche de SiC (11, 11').

14. Partie de connecteur mâle ou femelle selon la revendication 13,
**caractérisée en ce que** le corps d'électrode (5), la liaison électrique ainsi que la couche d'or (12) du système de corps d'électrode sont reliés en une seule pièce et sont composés d'or.
